# EUROPÄISCHE PATENTANMELDUNG

(11) **EP 0 774 457 A1**
(43) Veröffentlichungstag der Anmeldung: **21.05.1997**
(21) Anmeldenummer: 96117821.7
(22) Anmeldetag: 07.11.1996
(51) Int. Cl.: C07C 279/22, A61K 31/155

(54) **Substituierte Benzoldicarbonsäure-diguanidide, Verfahren zu ihrer Herstellung, ihre Verwendung als Medikament oder Diagnostikum sowie sie enthaltendes Medikament**

(30) Priorität: 20.11.1995 DE 19543194; 01.07.1996 DE 19626327
(71) Anmelder: HOECHST AKTIENGESELLSCHAFT, 65929 Frankfurt am Main (DE)
(72) Erfinder: Kleemann, Heinz-Werner, Dr., 65474 Bischofsheim (DE); Brendel, Joachim, Dr., 61118 Bad Vilbel (DE); Schwark, Jan-Robert, Dr., 65929 Frankfurt (DE); Weichert, Andreas, Dr., 63329 Egelsbach (DE); Lang, Hans Jochen, Dr., 65719 Hofheim (DE); Albus, Udo, Dr., 61197 Florstadt (DE); Scholz, Wolfgang, Dr., 65760 Eschborn (DE)

(57) **Zusammenfassung**

Benzoldicarbonsäure-diguanidide der Formel I 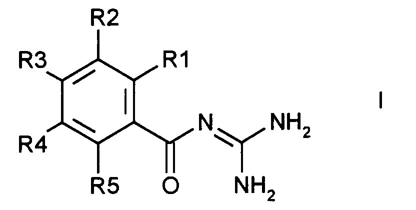 worin R(1) bis R(5) die in den Ansprüchen gegebenen Bedeutungen haben, sind wertvolle antiarrhythmische Arzneimittel mit cardioprotektiver Komponente, auch zur Prävention ischämisch induzierter Schäden, insbesondere bei der Auslösung ischämisch induzierter Herzarrhythmien. Sie werden infolge Inhibition des zellulären Na⁺/H⁺ -Austauschmechanismus zur Behandlung akuter oder chronischer durch Ischämie ausgelöster Schäden verwendet werden. Darüber hinaus zeichnen sie sich durch starke inhibierende Wirkung auf die Proliferationen von Zellen aus. Sie sind zur Verhinderung der Genese des Bluthochdrucks geeignet.

## Beschreibung

Die Erfindung betrifft Benzoldicarbonsäure-diguanidide der Formel I 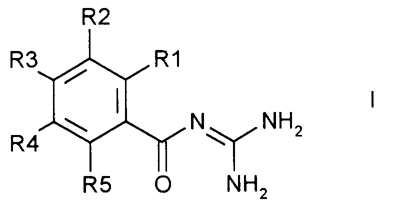 worin bedeuten:
einer der Reste R(1), R(2), R(3) und R(5)
   -CO-N=C(NH₂)₂;
und die jeweils anderen Reste R(1), R(2), R(3) und R(5):
   - R(1) und R(5): unabhängig voneinander Wasserstoff, Alkyl mit 1, 2, 3 oder 4 C-Atomen, F, Cl, -OR(32), -NR(33)R(34) oder CF₃;
   - R(32), R(33) und R(34): unabhängig voneinander Wasserstoff oder Alkyl mit 1, 2, 3 oder 4 C-Atomen;
   - R(2): Wasserstoff, F, Cl, Br, I, OH, -CN, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
   - m: Null, 1 oder 2;
   - R(14): -(C₃-C₈)-Cycloalkyl oder Phenyl,
   welches nicht substituiert oder substituiert ist mit 1 - 3 Substituenten ausgewählt aus der Gruppe bestehend aus F und Cl, -CF₃, Methyl, Methoxy und -NR(15)R(16);
   - R(15) und R(16): Wasserstoff oder -CH₃;
   oder
   - R(2): R(22)-SO₂-, R(23)R(24)N-CO-, R(28)-CO- oder R(29)R(30)N-SO₂-;
   - R(22) und R(28): unabhängig voneinander Methyl oder -CF₃;
   - R(23), R(24), R(29) und R(30): unabhängig voneinander Wasserstoff oder Methyl;
   oder
   - R(2): -OR(35) oder -NR(35)R(36);
   - R(35) und R(36): unabhängig voneinander Wasserstoff oder Alkyl mit 1, 2, 3, 4, 5 oder 6 C-Atomen;
   oder
   - R(35) und R(36): gemeinsam 4 - 7 Methylengruppen, von denen eine CH₂-Gruppe durch Sauerstoff, -S-, -NH-, -NCH₃ oder -N-Benzyl ersetzt sein kann;
   - R(3): Wasserstoff, -SR(25), -OR(25), -NR(25)R(26), -CR(25)R(26)R(27);
   - R(25): Wasserstoff, Alkyl mit 1, 2, 3, 4, 5, 6, 7 oder 8 C-Atomen oder Phenyl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   oder
   - R(25: -(C₁-C₉)-Heteroaryl,
   das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
   - R(26) und R(27): unabhängig voneinander wie R(25) definiert oder Wasserstoff oder Alkyl mit 1, 2, 3, 4, 5, 6, 7 oder 8 C-Atomen;
   - R(4): CF₃, Alkyl mit 1, 2, 3, 4, 5, 6, 7 oder 8 C-Atomen, Alkenyl mit 2, 3, 4, 5, 6, 7 oder 8 C-Atomen, -(C₃-C₈)-Cycloalkyl oder -(CH₂)ₘR(14);
   - m: 1 oder 2;
   - R(14): -(C₃-C₈)-Cycloalkyl oder Phenyl,
   welches nicht substituiert oder substituiert ist mit 1 - 3 Substituenten ausgewählt aus der Gruppe bestehend aus F und Cl, -CF₃, Methyl, Methoxy und -NR(15)R(16);
   - R(15) und R(16): Wasserstoff oder -CH₃;
   oder
   - R(4): Phenyl,
   welches substituiert ist mit 2, 3, 4 oder fünf Substituenten ausgewählt aus der Gruppe bestehend aus F, Cl, CF₃, Methyl, Methoxy und -NR(15)R(16);
   - R(15) und R(16): unabhängig voneinander Wasserstoff oder CH₃;
   sowie deren pharmazeutisch verträgliche Salze.

Bevorzugt sind Verbindungen der Formel I, in denen bedeuten:
einer der Reste R(1), R(2), R(3) und R(5)
   -CO-N=C(NH₂)₂;
und die jeweils anderen Reste R(1), R(2), R(3) und R(5)
   - R(1) und R(5): unabhängig voneinander Wasserstoff, Alkyl mit 1, 2, 3 oder 4 C-Atomen, F, Cl, -OR(32) oder -CF₃;
   - R(32): Wasserstoff oder Alkyl mit 1, 2, 3 oder 4 C-Atomen;
   - R(2): Wasserstoff, F, Cl, Br, I, OH, -CF₃, Alkyl mit 1, 2, 3 oder 4 C-Atomen, Alkenyl mit 2, 3 oder 4 C-Atomen oder -(CH₂)ₘR(14);
   - m: Null, 1 oder 2;
   - R(14): -(C₃-C₈)-Cycloalkyl oder Phenyl,
   welches nicht substituiert oder substituiert ist mit 1 - 2 Substituenten ausgewählt aus der Gruppe bestehend aus F und Cl, -CF₃, Methyl und Methoxy;
   oder
   - R(2): R(22)-SO₂-, R(23)R(24)N-CO-, R(28)-CO- oder R(29)R(30)N-SO₂-;
   - R(22) und R(28): unabhängig voneinander Methyl oder -CF₃;
   - R(23), R(24), R(29) und R(30): unabhängig voneinander Wasserstoff oder Methyl;
   oder
   - R(2): -OR(35) oder -NR(35)R(36);
   - R(35) und R(36): unabhängig voneinander Wasserstoff, Methyl oder Ethyl; oder
   - R(35) und R(36): gemeinsam 4 - 5 Methylengruppen, von denen eine CH₂-Gruppe durch Sauerstoff, -S-, -NH- oder -NCH₃ ersetzt sein kann;
   - R(3): Wasserstoff, -SR(25), -OR(25), -NR(25)R(26) oder -CR(25)R(26)R(27);
   - R(25): Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, CF₃, CH₃, Methoxy und Dimethylamino;
   oder
   - R(25): -(C₁-C₉)-Heteroaryl,
   das unsubstituiert oder substituiert ist mit 1 - 2 Substituenten ausgewählt aus der Gruppe bestehend aus F, Cl, -CF₃, CH₃, Methoxy und Dimethylamino;
   - R(26) und R(27): unabhängig voneinander Wasserstoff oder Alkyl mit 1, 2, 3 oder 4 C-Atomen;
   - R(4): -CF₃, Alkyl mit 1, 2, 3, 4, 5 oder 6 C-Atomen, Alkenyl mit 2, 3, 4, 5 oder 6 C-Atomen, -(C₅-C₆)-Cycloalkyl oder -CH₂R(14);
   - R(14): -(C₅-C₆)-Cycloalkyl oder Phenyl,
   welches nicht substituiert oder substituiert ist mit 1 - 2 Substituenten ausgewählt aus der Gruppe bestehend aus F und Cl, -CF₃, Methyl und Methoxy;
   oder
   - R(4): Phenyl,
   welches substituiert ist mit 2, 3, 4 oder fünf Substituenten ausgewählt aus der Gruppe bestehend aus F, Cl, CF₃, Methyl und Methoxy;
   sowie deren pharmazeutisch verträgliche Salze.

Besonders bevorzugt sind Verbindungen der Formel I, in denen bedeuten:
einer der Reste R(1), R(2), R(3) und R(5)
   -CO-N=C(NH₂)₂;
und die jeweils anderen Reste R(1), R(2), R(3) und R(5):
   - R(1) und R(5): unabhängig voneinander Wasserstoff, Alkyl mit 1, 2, 3 oder 4 C-Atomen, F, Cl, OH, Methoxy oder -CF₃;
   - R(2): Wasserstoff, F, Cl, OH, -CF₃, Alkyl mit 1, 2, 3 oder 4 C-Atomen, Alkenyl mit 2, 3 oder 4 C-Atomen oder -(CH₂)ₘR(14);
   - m: Null, 1 oder 2;
   - R(14): -(C₃-C₈)-Cycloalkyl oder Phenyl,
   welches nicht substituiert oder substituiert ist mit 1 - 2 Substituenten ausgewählt aus der Gruppe bestehend aus F und Cl, -CF₃, Methyl und Methoxy;
   oder
   - R(2): R(22)-SO₂-, R(23)R(24)N-CO-, R(28)-CO- oder R(29)R(30)N-SO₂-;
   - R(22) und R(28): unabhängig voneinander Methyl oder -CF₃;
   - R(23), R(24), R(29) und R(30): unabhängig voneinander Wasserstoff oder Methyl;
   oder
   - R(2): -OR(35) oder -NR(35)R(36);
   - R(35) und R(36): unabhängig voneinander Wasserstoff, Methyl oder Ethyl;
   oder
   - R(35) und R(36): gemeinsam 4 - 5 Methylengruppen, von denen eine CH₂-Gruppe durch Sauerstoff, -S-, -NH- oder -NCH₃ ersetzt sein kann;
   - R(3): Wasserstoff, -SR(25), -OR(25), -NR(25)R(26) oder -CR(25)R(26)R(27);
   - R(25): Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, CF₃, CH₃, Methoxy und Dimethylamino;
   oder
   - R(25): -(C₁-C₉)-Heteroaryl,
   das unsubstituiert oder substituiert ist mit 1 - 2 Substituenten ausgewählt aus der Gruppe bestehend aus F, Cl, CF₃, CH₃, Methoxy und Dimethylamino;
   - R(26) und R(27): unabhängig voneinander Wasserstoff oder Alkyl mit 1, 2, 3 oder 4 C-Atomen;
   - R(4): CF₃, Alkyl mit 1, 2, 3, 4, 5 oder 6 C-Atomen, Alkenyl mit 2, 3, 4, 5 oder 6 C-Atomen, -(C₅-C₆)-Cycloalkyl oder -CH₂R(14);
   - R(14): -(C₅-C₆)-Cycloalkyl oder Phenyl,
   welches nicht substituiert oder substituiert ist mit 1 - 2 Substituenten ausgewählt aus der Gruppe bestehend aus F und Cl, -CF₃, Methyl und Methoxy;
   oder
   - R(4): Phenyl,
   welches substituiert ist mit 2, 3, 4 oder fünf Substituenten ausgewählt aus der Gruppe bestehend aus F, Cl, CF₃, Methyl und Methoxy;
   sowie deren pharmazeutisch verträgliche Salze.

Ganz besonders bevorzugt sind Verbindungen der Formel I, in denen bedeuten:
einer der Reste R(1), R(2), R(3) und R(5)
   -CO-N=C(NH₂)₂;
und die jeweils anderen Reste R(1), R(2), R(3) und R(5):
   - R(1) und R(5): unabhängig voneinander Wasserstoff, Alkyl mit 1, 2, 3 oder 4 C-Atomen, F, Cl, OH, Methoxy oder -CF₃;
   - R(2): Wasserstoff, F, Cl, OH, -CF₃, Alkyl mit 1, 2, 3 oder 4 C-Atomen oder -(CH₂)ₘR(14);
   - m: Null, 1 oder 2;
   - R(14): -(C₅-C₆)-Cycloalkyl oder Phenyl,
   welches nicht substituiert oder substituiert ist mit 1 - 2 Substituenten ausgewählt aus der Gruppe bestehend aus F und Cl, -CF₃, Methyl und Methoxy;
   oder
   - R(2): -OR(35) oder -NR(35)R(36);
   - R(35) und R(36): unabhängig voneinander Wasserstoff, Methyl oder Ethyl;
   oder
   - R(35) und R(36): gemeinsam 4 - 5 Methylengruppen, von denen eine CH₂-Gruppe durch Sauerstoff, -S-, -NH- oder -NCH₃ ersetzt sein kann;
   - R(3): Wasserstoff oder -OR(25);
   - R(25): Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, CF₃, CH₃ und Methoxy;
   oder
   - R(25): -(C₁-C₉)-Heteroaryl,
   das unsubstituiert oder substituiert ist mit 1-2 Substituenten aus der Gruppe F, Cl, -CF₃, CH₃ und Methoxy;
   - R(4): CF₃, Alkyl mit 1, 2, 3, 4, 5 oder 6 C-Atomen, Alkenyl mit 2, 3, 4, 5 oder 6 C-Atomen, -(C₅-C₆)-Cycloalkyl oder -CH₂R(14);
   - R(14): -(C₅-C₆)-Cycloalkyl oder Phenyl,
   welches nicht substituiert oder substituiert ist mit 1 - 2 Substituenten ausgewählt aus der Gruppe bestehend aus F, Cl, -CF₃, Methyl und Methoxy;
   oder
   - R(4): Phenyl,
   welches substituiert ist mit 2, 3, 4 oder fünf Substituenten ausgewählt aus der Gruppe bestehend aus F, Cl, CF₃, Methyl und Methoxy;
   sowie deren pharmazeutisch verträgliche Salze.

Die bezeichneten Alkylreste können geradkettig oder verzweigt vorliegen.

Unter (C₁-C₉)-Heteroaryl werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(5) ein oder mehrere Asymmetriezentren, so können diese unabhängig voneinander sowohl S als auch R konfiguriert sein. Die Verbindungen können als optische Isomere, als Diastereomere, als Racemate oder als Gemische derselben vorliegen.

Die Erfindung betrifft weiterhin ein Verfahren zur Herstellung der Verbindungen 1, dadurch gekennzeichnet, daß man Verbindungen der Formel II 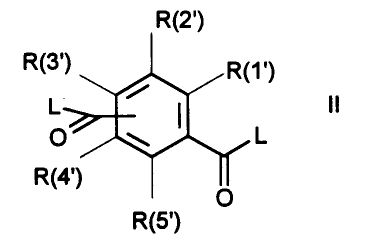 worin R(1') bis R(5') die oben für R(1) bis R(5) angegebenen Bedeutungen haben, von denen jedoch mindestens einer der Substituenten R(1') bis R(5') die gezeichnete COL-Gruppe ist, und worin L für leicht nucleophil substituierbare Fluchtgruppen steht,
mit Guanidin umsetzt.

Die aktivierten Säurederivate der Formel II, worin L eine Alkoxy-, vorzugsweise eine Methoxygruppe oder Phenoxygruppe, eine Phenylthio-, Methylthio-, 2-Pyridylthiogruppe, oder einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann. Neben den Carbonsäurechloriden der Formel II (L = Cl) lassen sich auch weitere aktivierte Säurederivate der Formel II in an sich bekannter Weise direkt aus den zugrundeliegenden Benzoldicarbonsäurederivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1,351-367 (1962)], die gemischten Anhydride II mit Cl-COOC₂H₅ oder Tosylchlorid in Gegenwart von Triethylamin in einem inerten Lösungsmittel, wie auch die Aktivierungen von Benzoldicarbonsäuren mit Dicyclohexylcarbodiimid (DCC) oder mit O-[(Cyano(ethoxycarbonyl)methylen)amino]-1,1,3,3-tetramethyluronium-tetrafluoroborat] ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der allgemeinen Formel II sind unter Angabe von Quellenliteratur in J. March, Advanced Organic Chemistry, Third Edition (John Wiley & Sons, 1985), S. 350 angegeben. Die Umsetzung eines aktivierten Carbonsäurederivates der Formel I mit Guanidin erfolgt in an sich bekannter Weise in einem protischen oder aprotischen polaren aber inerten organischen Lösungsmittel. Dabei haben sich bei der Umsetzung der Benzoldicarbonsäuremethylester (II, L = OMe) mit Guanidin Methanol, Isopropanol oder THF zwischen 20°C und Siedetemperatur dieser Lösungsmittel bewährt. Bei den meisten Umsetzungen von Verbindungen II mit salzfreien Guanidin wurde vorteilhaft in inerten Lösungsmitteln wie THF, Dimethoxyethan, Dioxan oder Isopropanol gearbeitet. Aber auch Wasser kann als Lösungsmittel dienen.

Wenn L = Cl bedeutet, arbeitet man vorteilhaft unter Zusatz eines Säurefängers, z.B. in Form von überschüssigen Guanidin zur Abbindung der Halogenwasserstoffsäure.
Die Einführung der im Phenylteil mit Schwefel-, Sauerstoff- oder Stickstoffnucleophilen substituierten Verbindungen gelingt durch literaturbekannte Methoden der nucleophilen Substitution an Derivaten der Benzoldicarbonsäure-dialkylester. Als Abgangsgruppe am Benzoldicarbonsäurederivat haben sich bei dieser Substitution Halogenide und Trifluormethansulfonate bewährt. Man arbeitet vorteilhaft in einem dipolar aprotischen Lösungsmittel, wie DMF oder TMU, bei einer Temperatur von 0°C bis zum Siedepunkt des Lösungsmittels, bevorzugt von 80°C bis zum Siedepunkt des Lösungsmittels. Als Säurefänger dient vorteilhaft ein Alkali- oder Erdalkalisalz mit einem Anion hoher Basizität und geringer Nucleophilie, zum Beispiel K₂CO₃ oder CsCO₃.

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Benzoldicarbonsäure-diguanidide I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Ascorbate, Lactate, Sulfate, Citrate, Tartrate, Acetate, Phosphate, Methylsulfonate, p-Toluolsulfonate.

In der US-Patentschrift 5 091 394 (HOE 89/F 288) und in der europäischen Offenlegungsschrift 0 556 674 (HOE 92/F 034) werden Benzoylguanidine, nicht jedoch Benzoldicarbonsäure-diguanidide beschrieben. Aus der WO 94/26709 ist ein Benzoylguanidin bekannt, welches in 5-Stellung einen Nitrophenyl-Substituenten enthält. Mehrfach substituierte 5-Phenyl-benzoylguanidine sind daraus jedoch weder bekannt, noch nahegelegt.

Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺ -Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden. Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Gegenüber den meisten bekannten Verbindungen weisen die Verbindungen nach der Erfindung eine signifikant verbesserte Wasserlöslichkeit auf. Daher sind sie wesentlich besser für i.v.-Applikation geeignet.

Die erfindungsgemäßen Verbindungen zeichnen sich gegenüber den bekannten gut wasserlöslichen Verbindungen durch ihre bessere Bioverfügbarkeit und Pharmakokinetik aus.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.
Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg Körpergewicht, vorzugsweise mindestens 0,01 mg/kg Körpergewicht, bis höchstens 10 mg/kg Körpergewicht, vorzugsweise bis höchstens 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

### Liste der Abkürzungen:

- Bn: Benzyl
- Brine: gesättigte wäßrige NaCl-Lösung
- CH₂Cl₂: Dichlormethan
- DCI: Desorption-Chemical Ionisation
- DIP: Diisopropylether
- DMA: Dimethylacetamid
- DME: Dimethoxyethan
- DMF: N,N-Dimethylformamid
- EE: Ethylacetat (EtOAc)
- EI: electron impact
- eq: Äquivalent
- ES: Elektrospray-Ionisation
- Et: Ethyl
- FAB: Fast Atom Bombardment
- HEP: n-Heptan
- HOAc: Essigsäure
- Me: Methyl
- MeOH: Methanol
- mp: Schmelzpunkt
- MTB: Methyltertiärbutylether
- NBS: N-Bromsuccinimid
- NMP: N-Methylpyrrolidon
- RT: Raumtemperatur
- THF: Tetrahydrofuran
- TMU: N,N,N',N'-Tetramethylharnstoff
- Tol: Toluol
- ZNS: Zentralnervensystem

### Experimenteller Teil

Allgemeine Vorschrift zur Herstellung von Benzoldicarbonsäure-diguanididen (I) aus Benzoldicarbonsäure-dialkylestern (II, L = O-Alkyl)

5 mmol des Benzoldicarbonsäure-dialkylesters der Formel II sowie 50 mmol Guanidin (freie Base) werden in 5 ml Isopropanol gelöst und bis zum vollständigen Umsatz (Dünnschichtkontrolle) unter Rückfluß gekocht (typische Reaktionszeit 5 Minuten bis 5 h). Anschließend wird mit 150 ml Wasser verdünnt und das Produkt abgesaugt. Gegebenenfalls wird an Kieselgel mit einem geeigneten Laufmittel, z. B. EE/MeOH 5 : 1 chromatographiert.

### Beispiel 1: 5-t-Butyl-isophthalsäure-diguanidid

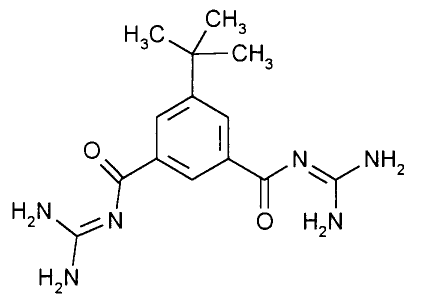

2.9 g 5*-t-*Butyl-isophthalsäure-dimethylester werden nach der allgemeinen Vorschrift zur Herstellung von Benzoldicarbonsäure-diguanididen (I) umgesetzt (Reaktionszeit 10 Minuten). Man erhält 2.7 g weißer Kristalle, mp > 270°C.
R_{f} (Aceton/Wasser 10:1) = 0.13 MS (ES) : 305 (M + H)⁺

### Beispiel 2: 5-[3,5-Bis(trifluormethyl)phenyl]-isophthalsäure-diguanidid

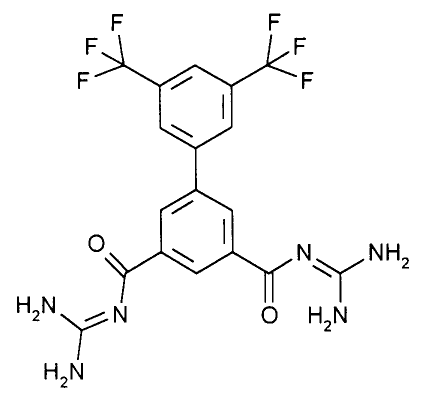

600 mg 5-[3,5-Bis(trifluormethyl)phenyl]-isophthalsäure-dimethyester werden nach der allgemeinen Vorschrift zur Herstellung von Benzoldicarbonsäure-diguanididen (I) umgesetzt (Reaktionszeit 10 Minuten). Man erhält 580 mg weißer Kristalle, mp 248 ^{o}C unter Zersetzung.
R_{f} (Aceton/Wasser 10:1) = 0.21 MS (FAB) : 461 (M + H)⁺

### 2 a: 5-(3,5-Bis(trifluormethyl)phenyl]-isophthalsäure-dimethyester

3.73 g 5-Brom-isophthalsäure-dimethyester, 2.74 g 3,5-Bis(trifluormethyl)phenylboronsäure, 2.1 g Na₂CO₃, 225 mg Pd(II)-acetat und 525 mg Triphenylphosphin werden in 100 ml Toluol und 20 ml Wasser 3 h bei 100 ^{o}C gerührt. Man läßt abkühlen, verdünnt mit 200 ml EE und extrahiert 2 mal mit je 100 ml gesättigter wäßriger NaHCO₃-Lösung. Über Na₂SO₄ wird getrocknet und das Solvens im Vakuum entfernt. Chromatographie an Kieselgel mit EE/HEP 1:8 liefert 1.3g eines hellgelben Feststoffs, mp 151 ^{o}C.
R_{f} (EE/HEP 1:8) = 0.21 MS (FAB) : 407 (M + H)⁺

Die Titelverbindungen der Beispiele 3 - 5 werden analog Beispiel 2 synthetisiert, die Dimethylester-Vorstufen werden analog 2 a erhalten.

### Beispiel 3: 5-(3,5-Dichlorphenyl)-isophthalsäure-diguanidid

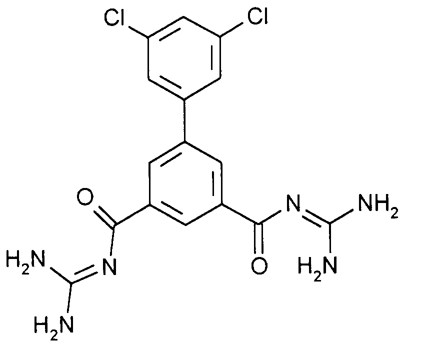

mp 250°C unter Zersetzung
R_{f} (Aceton/Wasser 10:1) = 0.13 MS (ES) : 393 (M + H)⁺

### Beispiel 4: 5-(2,4-Dichlorphenyl)-isophthalsäure- diguanidid

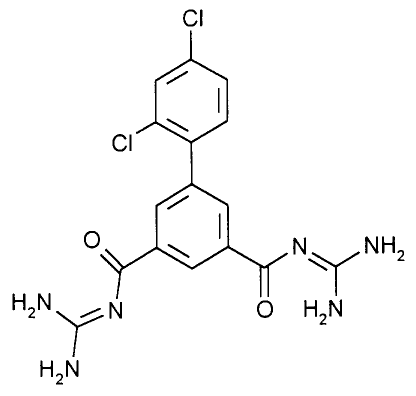

mp > 250°C unter Zersetzung
R_{f} (Aceton/Wasser 10:1) = 0.15 MS (ES) : 393 (M + H)⁺

### Beispiel 5: 5-(3-Chlor-4-fluorphenyl)-isophthalsäure- diguanidid

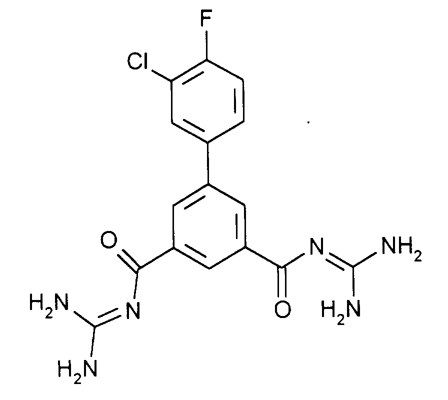

mp 231°C unter Zersetzung
R_{f} (Aceton/Wasser 10:1) = 0.14 MS (ES) : 377 (M + H)⁺

### Beispiel 6: 5-Cyclohexyl-isophthalsäure-diguanidid

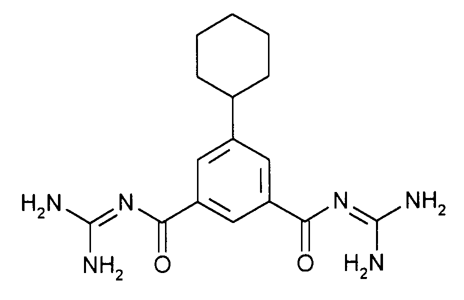

### a) 5-Cyclohexyl-isophthalsäure-dimethylester

Zu 100 ml einer Lösung 0.5 M ZnCl₂-Lösung in THF werden 22 ml einer 2 M Lösung von Cyclohexylmagnesiumchlorid in Diethylether zugetropft. 5 h wird bei 50°C gerührt und die zinkorganische Verbindung als Lösung A direkt weiter umgesetzt.
3.9 g 5-Isophthalsäure-dimethylester` 420 mg [1,1-Bis(diphenylphosphino)ferrocen]Pd(II)-chlorid und 130 mg CuI werden in 70 ml THF suspendiert. Bei RT wird dann Lösung A zugetropft und 8 h bei dieser Temperatur gerührt. Das Reaktionsgemisch wird auf 300 ml gesättigter wäßriger NaHCO₃-Lösung gegossen, mit 100 ml Wasser verdünnt, der Niederschlag abfiltriert und 4 mal mit je 200 ml EE extrahiert. Über Na₂SO₄ wird getrocknet und das Solvens im Vakuum entfernt. Chromatographie an Kieselgel mit EE/HEP 1:4 liefert 2.7 g eines farblosen Öls.
R_{f} (EE/HEP 1:4) = 0.40 MS (FAB) : 277 (M + H)⁺

### b) 5-Cyclohexyl-isophthalsäure-diguanidid

5.3 g Guanidin-Hydrochlorid werden in 55 ml DMF gelöst und eine Lösung von 5.6 g Kalium-tert.-butylat gelöst in 50 ml DMF bei RT zugetropft. Anschließend wird 2 h bei RT gerührt, dann eine Lösung von 1.4 g 5-Cyclohexylisophthalsäure-dimethylester in 15 ml DMF zugetropft und 24 h bei RT gerührt. Das Reaktionsgemisch wird auf 1 l Wasser gegossen und 3 mal mit je 100 ml EE extrahiert. Über Na₂SO₄ wird getrocknet und das Solvens im Vakuum entfernt. Der Rückstand wird in 50 ml EE suspendiert und mit einer gesättigten Lösung von Maleinsäure in EE auf pH = 2-3 eingestellt. Der kristalline Rückstand wird abfiltriert, mit 50 ml EE gewaschen und anschließend in je 50 ml einer gesättigten wäßrigen Na₂CO₃-Lösung, einer gesättigten wäßrigen NaHCO₃-Lösung und Wasser gelöst und die freie Base durch Extrktion mit 3 mal je 100 ml EE isoliert. Über Na₂SO₄ wird getrocknet und das Solvens im Vakuum entfernt. Man erhält 1.1 g farbloser Kristalle,
mp 219 - 220°C.
R_{f} (Aceton/Wasser 10:1) = 0.23 MS (FAB) : 331 (M + H)⁺

### Pharmakologische Daten:

Inhibition des Na⁺/H⁺ -Exchangers von Kaninchenerythrocyten Weiße Neuseeland-Kaninchen (Ivanovas) erhielten eine Standard-Diät mit 2% Cholesterin für sechs Wochen, um den Na⁺/H⁺ -Austausch zu aktivieren und so den Na⁺ -Influx in die Erythrocyten via Na⁺/H⁺ -Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺ - Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37∠C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺ -Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺ -Exchangers: | |
|---|---|
| Beispiel | IC₅₀ mol/l |
| 1 | 0.15 |
| 3 | 0.2 |
| 5 | 0.2 |

## Patentansprüche

1. Benzoldicarbonsäure-diguanidide der Formel I 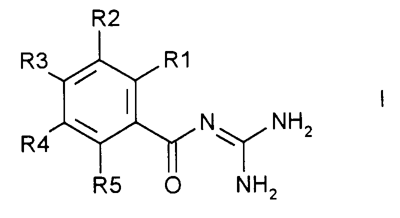 worin bedeuten:
einer der Reste R(1), R(2), R(3) und R(5)
-CO-N=C(NH₂)₂;
und die jeweils anderen Reste R(1), R(2), R(3) und R(5):
R(1) und R(5)
unabhängig voneinander Wasserstoff, Alkyl mit 1, 2, 3 oder 4 C-Atomen, F, Cl, -OR(32), -NR(33)R(34) oder CF₃;
R(32), R(33) und R(34)
unabhängig voneinander Wasserstoff oder Alkyl mit 1, 2, 3 oder 4 C-Atomen;
R(2) Wasserstoff, F, Cl, Br, I, OH, -C°N, CF₃, -CO-N=C(NH₂)₂, Alkyl mit 1, 2, 3, 4, 5, 6, 7 oder 8 C-Atomen, Alkenyl mit 2, 3, 4, 5, 6, 7 oder 8 C-Atomen oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
unabhängig voneinander Wasserstoff oder -CH₃;
oder
R(2) R(22)-SO₂-, R(23)R(24)N-CO-, R(28)-CO- oder R(29)R(30)N-SO₂;
R(22) und R(28)
unabhängig voneinander Methyl oder -CF₃;
R(23), R(24), R(29) und R(30)
unabhängig voneinander Wasserstoff oder Methyl;
oder
R(2) -OR(35) oder -NR(35)R(36);
R(35) und R(36)
unabhängig voneinander Wasserstoff oder Alkyl mit 1, 2, 3, 4, 5 oder 6 C-Atomen;
oder
R(35) und R(36)
gemeinsam 4 - 7 Methylengruppen, von denen eine CH₂-Gruppe durch Sauerstoff, -S-, -NH-, -NCH₃ oder -N-Benzyl ersetzt sein kann;
R(3) Wasserstoff, -SR(25), -OR(25), -NR(25)R(26), -CR(25)R(26)R(27);
R(25) Wasserstoff, Alkyl mit 1, 2, 3, 4, 5, 6, 7 oder 8 C-Atomen oder Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(25 -(C₁-C₉)-Heteroaryl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
R(26) und R(27)
unabhängig voneinander wie R(25) definiert oder Wasserstoff oder Alkyl mit 1, 2, 3, 4, 5, 6, 7 oder 8 C-Atomen;
R(4) CF₃, Alkyl mit 1, 2, 3, 4, 5, 6, 7 oder 8 C-Atomen, Alkenyl mit 2, 3, 4, 5, 6, 7 oder 8 C-Atomen, -(C₃-C₈)-Cycloalkyl oder -(CH₂)ₘR(14);
m 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
unabhängig voneinander Wasserstoff oder -CH₃;
oder
R(4) Phenyl,
welches substituiert ist mit 2, 3, 4 oder fünf Substituenten ausgewählt aus der Gruppe bestehend aus F, Cl, CF₃, Methyl, Methoxy und -NR(15)R(16);
R(15) und R(16)
unabhängig voneinander Wasserstoff oder CH₃;
sowie deren pharmazeutisch verträgliche Salze.

2. Verbindung der Formel I nach Anspruch 1, in der bedeuten:
einer der Reste R(1), R(2), R(3) und R(5)
-CO-N=C(NH₂)₂;
und die jeweils anderen Reste R(1), R(2), R(3) und R(5):
R(1) und R(5)
unabhängig voneinander Wasserstoff, Alkyl mit 1, 2, 3 oder 4 C-Atomen, F, Cl, -OR(32) oder CF₃;
R(32) Wasserstoff oder Alkyl mit 1, 2, 3 oder 4 C-Atomen;
R(2) Wasserstoff, F, Cl, Br, I, OH, CF₃, Alkyl mit 1, 2, 3 oder 4 C-Atomen, Alkenyl mit 2, 3 oder 4 C-Atomen oder -(CH₂)ₘR(14);
m Null, 1 oder 2;
R(14) -(C₃-C₈)-Cycloalkyl oder Phenyl,
welches nicht substituiert oder substituiert ist mit 1 - 2 Substituenten ausgewählt aus der Gruppe bestehend aus F und Cl, -CF₃, Methyl und Methoxy;
oder
R(2) R(22)-SO₂-, R(23)R(24)N-CO-, R(28)-CO- oder R(29)R(30)N-SO₂;
R(22) und R(28)
unabhängig voneinander Methyl oder -CF₃;
R(23), R(24), R(29) und R(30)
unabhängig voneinander Wasserstoff oder Methyl;
oder
R(2) -OR(35) oder -NR(35)R(36);
R(35) und R(36)
unabhängig voneinander Wasserstoff, Methyl oder Ethyl;
oder
R(35) und R(36)
gemeinsam 4 - 5 Methylengruppen, von denen eine CH₂-Gruppe durch Sauerstoff, -S-, -NH- oder -NCH₃ ersetzt sein kann;
R(3) Wasserstoff, -SR(25), -OR(25), -NR(25)R(26) oder -CR(25)R(26)R(27);
R(25) Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, CF₃, CH₃, Methoxy und Dimethylamino;
oder
R(25) -(C₁-C₉)-Heteroaryl,
das unsubstituiert oder substituiert ist mit 1 - 2 Substituenten ausgewählt aus der Gruppe bestehend aus F, Cl, CF₃, CH₃, Methoxy und Dimethylamino;
R(26) und R(27)
unabhängig voneinander Wasserstoff oder Alkyl mit 1, 2, 3 oder 4 C-Atomen;
R(4) CF₃, Alkyl mit 1, 2, 3, 4, 5 oder 6 C-Atomen, Alkenyl mit 2, 3, 4, 5 oder 6 C-Atomen, -(C₅-C₆)-Cycloalkyl oder -CH₂R(14);
R(14) -(C₅-C₆)-Cycloalkyl oder Phenyl,
welches nicht substituiert oder substituiert ist mit 1 - 2 Substituenten ausgewählt aus der Gruppe bestehend aus F und Cl, -CF₃, Methyl und Methoxy;
oder
R(4) Phenyl,
welches substituiert ist mit 2, 3, 4 oder fünf Substituenten ausgewählt aus der Gruppe bestehend aus F, Cl, CF₃, Methyl und Methoxy;
sowie deren pharmazeutisch verträgliche Salze.

3. Verbindungen der Formel I nach Anspruch 1 oder 2, dadurch gekennzeichnet, daß darin bedeuten:
einer der Reste R(1), R(2), R(3) und R(5)
-CO-N=C(NH₂)₂;
und die jeweils anderen Reste R(1), R(2), R(3) und R(5):
R(1) und R(5)
unabhängig voneinander Wasserstoff, Alkyl mit 1, 2, 3 oder 4 C-Atomen, F, Cl, OH, Methoxy oder CF₃;
R(2) Wasserstoff, F, Cl, OH, CF₃, Alkyl mit 1, 2, 3 oder 4 C-Atomen, Alkenyl mit 2, 3 oder 4 C-Atomen oder -(CH₂)ₘR(14);
m Null, 1 oder 2;
R(14) -(C₃-C₈)-Cycloalkyl oder Phenyl,
welches nicht substituiert oder substituiert ist mit 1 - 2 Substituenten ausgewählt aus der Gruppe bestehend aus F und Cl, -CF₃, Methyl und Methoxy;
oder
R(2) R(22)-SO₂-, R(23)R(24)N-CO-, R(28)-CO- oder R(29)R(30)N-SO₂;
R(22) und R(28)
unabhängig voneinander Methyl oder -CF₃;
R(23), R(24), R(29) und R(30)
unabhängig voneinander Wasserstoff oder Methyl;
oder
R(2) -OR(35) oder -NR(35)R(36);
R(35) und R(36)
unabhängig voneinander Wasserstoff, Methyl oder Ethyl;
oder
R(35) und R(36)
gemeinsam 4 - 5 Methylengruppen, von denen eine CH₂-Gruppe durch Sauerstoff, -S-, -NH- oder -NCH₃ ersetzt sein kann;
R(3) Wasserstoff, -SR(25), -OR(25), -NR(25)R(26) oder -CR(25)R(26)R(27);
R(25) Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, CF₃, CH₃, Methoxy und Dimethylamino;
oder
R(25) -(C₁-C₉)-Heteroaryl,
das unsubstituiert oder substituiert ist mit 1 - 2 Substituenten ausgewählt aus der Gruppe bestehend aus F, Cl, CF₃, CH₃, Methoxy und Dimethylamino;
R(26) und R(27)
unabhängig voneinander Wasserstoff oder Alkyl mit 1, 2, 3 oder 4 C-Atomen;
R(4) CF₃, Alkyl mit 1, 2, 3, 4, 5 oder 6 C-Atomen, Alkenyl mit 2, 3, 4, 5 oder 6 C-Atomen, -(C₅-C₆)-Cycloalkyl oder -CH₂R(14);
R(14) -(C₅-C₆)-Cycloalkyl oder Phenyl,
welches nicht substituiert oder substituiert ist mit 1 - 2 Substituenten ausgewählt aus der Gruppe bestehend aus F und Cl, -CF₃, Methyl und Methoxy;
oder
R(4) Phenyl,
welches substituiert ist mit 2, 3, 4 oder fünf Substituenten ausgewählt aus der Gruppe bestehend aus F, Cl, CF₃, Methyl und Methoxy.

4. Verbindung der Formel I nach Ansprüchen 1 bis 3, in der bedeuten:
einer der Reste R(1), R(2), R(3) und R(5)
-CO-N=C(NH₂)₂;
und die jeweils anderen Reste R(1), R(2), R(3) und R(5):
R(1) und R(5)
unabhängig voneinander Wasserstoff, Alkyl mit 1, 2, 3 oder 4 C-Atomen, F, Cl, OH, Methoxy oder CF₃;
R(2) Wasserstoff, F, Cl, OH, CF₃, Alkyl mit 1, 2, 3 oder 4 C-Atomen oder -(CH₂)ₘR(14);
m Null, 1 oder 2;
R(14) -(C₅-C₆)-Cycloalkyl oder Phenyl,
welches nicht substituiert oder substituiert ist mit 1 - 2 Substituenten ausgewählt aus der Gruppe bestehend aus F und Cl, -CF₃, Methyl und Methoxy;
oder
R(2) -OR(35) oder -NR(35)R(36);
R(35) und R(36)
unabhängig voneinander Wasserstoff, Methyl oder Ethyl;
oder
R(35) und R(36)
gemeinsam 4 - 5 Methylengruppen, von denen eine CH₂-Gruppe durch Sauerstoff, -S-, -NH- oder -NCH₃ ersetzt sein kann;
R(3) Wasserstoff oder -OR(25);
R(25) Wasserstoff, Alkyl mit 1, 2, 3 oder 4 C-Atomen oder Phenyl, das unsubstituiert oder substituiert ist mit 1 - 2 Substituenten ausgewählt aus der Gruppe bestehend aus F, Cl, CF₃, CH₃ und Methoxy;
oder
R(25) -(C₁-C₉)-Heteroaryl,
das unsubstituiert oder substituiert ist mit 1-2 Substituenten aus der Gruppe F, Cl, CF₃, CH₃ und Methoxy;
R(4) CF₃, Alkyl mit 1, 2, 3, 4, 5 oder 6 C-Atomen, Alkenyl mit 2, 3, 4, 5 oder 6 C-Atomen, -(C₅-C₆)-Cycloalkyl oder -CH₂R(14);
R(14) -(C₅-C₆)-Cycloalkyl oder Phenyl,
welches nicht substituiert oder substituiert ist mit 1 - 2 Substituenten ausgewählt aus der Gruppe bestehend aus F, Cl, -CF₃, Methyl und Methoxy;
oder
R(4) Phenyl,
welches substituiert ist mit 2 oder 3 Substituenten ausgewählt aus der Gruppe bestehend aus F, Cl, CF₃, Methyl und Methoxy.

5. Verfahren zur Herstellung einer Verbindung I nach Anspruch 1, dadurch gekennzeichnet, daß man eine Verbindung der Formel II 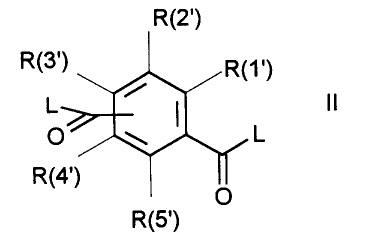 worin R(1) bis R(5) die oben angegebenen Bedeutungen haben und L für leicht nucleophil substituierbare Fluchtgruppen steht,
mit Guanidin umsetzt.

6. Verwendung einer Verbindung I nach Anspruch 1 zur Herstellung eines Medikaments zu Behandlung oder Prophylaxe von durch ischämische Zustände verursachten Krankheiten.

7. Methode zum Behandeln und zur Prophylaxe von durch ischämische Zustände verursachten Krankheiten, dadurch gekennzeichnet, daß man eine wirksame Menge einer Verbindung I nach Anspruch 1 mit den üblichen Zusatzstoffen versetzt und in einer geeigneten Darreichungsform verabreicht.

8. Verwendung einer Verbindung I nach Anspruch 1 zur Herstellung eines Medikaments zur Behandlung oder Prophylaxe des Herzinfarkts und von Arrhythmien.

9. Verwendung einer Verbindung I nach Anspruch 1 zur Herstellung eines Medikaments zur Behandlung oder Prophylaxe der Angina Pectoris.

10. Verwendung einer Verbindung I nach Anspruch 1 zur Herstellung eines Medikaments zur Behandlung oder Prophylaxe von ischämischen Zuständen des Herzens.

11. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

12. Verwendung einer Verbindung I nach Anspruch 1 zur Herstellung eines Medikaments zur Behandlung oder Prophylaxe von ischämischen Zuständen peripherer Organe und Gliedmaßen.

13. Verwendung einer Verbindung I nach Anspruch 1 zur Herstellung eines Medikaments zur Behandlung von Schockzuständen.

14. Verwendung einer Verbindung I nach Anspruch 1 zur Herstellung eines Medikaments zum Einsatz bei chirurgischen Operationen und Organtransplantationen.

15. Verwendung einer Verbindung I nach Anspruch 1 zur Herstellung eines Medikaments zur Konservierung und Lagerung von Transplantaten für chirurgische Maßnahmen.

16. Verwendung einer Verbindung I nach Anspruch I zur Herstellung eines Medikaments zur Behandlung von Krankheiten, bei denen die Zellproliferation eine primäre oder sekundäre Ursache darstellt, und somit ihre Verwendung als Antiatherosklerotika, Mittel gegen diabetische Spätkomplikationen, Krebserkrankungen, fibrotische Erkrankungen wie Lungenfibrose, Leberfibrose oder Nierenfibrose, Prostatahyperplasie.

17. Verwendung einer Verbindung I nach Anspruch 1 zur Herstellung eines wissenschaftliches Tools zur Inhibition des Na⁺/H⁺ -Exchangers, zur Diagnose der Hypertonie und proliferativer Erkrankungen.

18. Arzneimittel, enthaltend eine wirksame Menge einer Verbindung I nach einem oder mehreren der Ansprüche 1 bis 4.
